# EUROPEAN PATENT SPECIFICATION

(11) **EP 2 412 339 B1**
(45) Date of publication and mention of the grant of the patent: **08.10.2014**
(21) Application number: 11187048.1
(22) Date of filing: 09.10.2007
(51) Int. Cl.: A61F 2/38

(54) **Mobile/fixed prosthetic knee systems**
Mobile/fixierte Knieprothesensysteme
Systèmes de genou prothétique mobile et fixe

(30) Priority: 13.10.2006 US 829430 P; 13.10.2006 US 829432 P; 21.09.2007 US 859454
(43) Date of publication of application: 01.02.2012
(62) Divisional of application: 07844021.1
(73) Proprietor: DePuy (Ireland), County Cork (IE)
(72) Inventor: Hazebrouck, Stephen A, Winona Lake, IN Indiana 46590 (US)
(74) Representative: Belcher, Simon James

(56) References cited:
- WO-A1-2004/069105
- FR-A1- 2 698 536
- US-A- 4 353 136
- US-A- 5 282 868
- US-A1- 2006 015 185

## Description

The present invention relates to a knee prosthesis. Specifically, the invention relates to the tibial and bearing components of a knee prosthesis.

Movement (e.g. flexion and extension) of the natural human knee involves movements of the femur and the tibia. Specifically, during flexion and extension, the distal end of the femur and the proximal end of the tibia articulate relative to one another through a series of complex movements. Damage (e.g. trauma) or disease can deteriorate the bones, articular cartilage, and ligaments of the knee, which can ultimately affect the ability of the natural knee to function in such a manner. As a result, knee prostheses have been developed and implanted into surgically prepared ends of the femur and tibia.

A typical knee prosthesis for a total knee replacement, for example, includes a tibial component or tibial tray coupled to the patient's tibia, a femoral component coupled to the patient's femur, and a bearing component (or tibial insert) positioned between the tibial tray and the femoral component and including a bearing surface to accommodate the condyles of the femoral component. In some situations, it may be desirable that the tibial insert rotate relative to the tibial tray. Such rotation more closely replicates the motion of the patient's natural anatomy. In other cases, however, it may be desirable to prevent the tibial insert from rotating relative to the tibial tray. For example, various ligaments which support the knee may be compromised or damaged. In such a case, rotation of the tibial insert relative to the tibial tray may create an unstable knee. As such, a surgeon will decide on a case-by-case basis whether to use a rotating or non-rotating tibial assembly. This decision may be made pre-operatively or intra-operatively, for example. Additionally, it may be desirable to change a rotating tibial insert to a non-rotating tibial insert during a revision type surgery, for example.

FR-2698536 discloses a knee joint prosthesis which comprises a tibial tray and a tibial insert. The tray has a pair of curved grooves in its upper surface. The insert has a pair of curved rails in its lower surface which fit in the grooves when the insert is positioned on the tray. The insert rotates relative to the tray as it slides on the tray.

US-5282868 discloses a knee joint prosthesis which comprises a tibial plateau component having a pair of guide tracks formed in it. Respective condyle components are connected to one another and have downwardly extending ribs which are received in the guide tracks. The ribs can slide in the guide tracks so that the condyle components can slide on the plateau component, the sliding motion involving translation and rotation.

US-4353136 discloses a knee joint prosthesis in which the tibial component has a C-shaped arcuate groove formed in it. A meniscal component has a pair of ribs on its lower surface which are received in the groove in the tibial component. The meniscal component is able to slide on the tibial component, allowing rotation about the longitudinal axis of the leg.

The present invention provides an orthopaedic prosthesis as defined in claim 1.

In some embodiments, the first tab and the second tab may be integral to the tibial insert. In other embodiments, the first tab and the second tab may be removably coupled to the tibial insert. For example, in some embodiments, the tibial insert may include a third slot and a fourth slot defined in the bottom surface. In such embodiments, the first tab may have a first end received in the third slot of the tibial insert and a second end received in the first slot of the tibial tray. Additionally, the second tab may include a first end received in the fourth slot of the tibial insert and a second end received in the second slot of the tibial tray. In embodiments in which the first and second tab are removable from the tibial insert, the tibial insert may be rotatable relative to the tibial tray when the first tab and second tab are removed from the tibial insert.

In some embodiments, the first tab may include a first flange defined at the first end and a second flange defined at the second end and the second tab may include a first flange defined at the first end and a second flange defined at the second end. The first flange of the first tab may be received in the third slot and the second flange of the first tab may be received in the first slot. Similarly, the first flange of the second tab being may be received in the fourth slot and the second flange of the second tab being may be received in the second slot.

In some embodiments, the first slot may be defined in the upper surface of the platform at an angle relative to the second slot. Additionally, in some embodiments, the tibial tray may include an opening defined in the upper surface of the platform. The tibial insert may include a stem extending downwardly from the bottom surface and received in the opening of the tibial tray. In such embodiments, the first slot and second slot may be connected to the opening.

In some embodiments, each of the first and second curved slots has a concave-shaped top profile.

The tibial insert may be configured to rotate with respect to the tibial tray less than 90 degrees.

The first curved tab may be configured to slide along an first arc within the first curved slot and the second curved tab may be configured to slide along a second arc within the second curved slot when the tibial tray is rotated relative to the tibial insert. Further, in some embodiments, the tibial insert may include a stem extending downwardly from the bottom surface. The tibial tray may include an opening defmed on the upper surface of the platform between the first slot and the second slot. In such embodiments, the stem of the tibial insert may be received in the opening.

### BRIEF DESCRIPTION OF THE DRAWINGS

The detailed description particularly refers to the following figures, in which:
FIG. 1 is a sectional view of a tibial tray and a tibial insert configured to be coupled to the tray;
FIG. 2 a bottom view of the rotating tibial insert of FIG. 1 including two curved rails;
FIG. 3 is a top view of the tibial tray of FIG. 1 including two guide tracks formed therein;
FIG. 4 is a sectional view of a tibial tray and a tibial insert coupled to the tray showing the tibial insert including an upper, polymer and a lower, metal base configured to be coupled to the metal tibial tray;
FIG. 5 is a top view of the tibial tray of FIG. 4 including a bearing system;
FIG. 6 is a top view of another tibial tray showing a plurality of curved openings;
FIG. 7 is an enlarged sectional view of an exemplary cross-sectional shape of the openings of FIG. 6;
FIG. 8 is an enlarged sectional view of an exemplary cross-sectional shape of the openings of FIG. 6;
FIG. 9 is an enlarged sectional view of an exemplary cross-sectional shape of the openings of FIG. 6;
FIG. 10 is a perspective view of a rotating tibial assembly including a track system to guide the rotating movement of the tibial insert relative to the tibial tray.

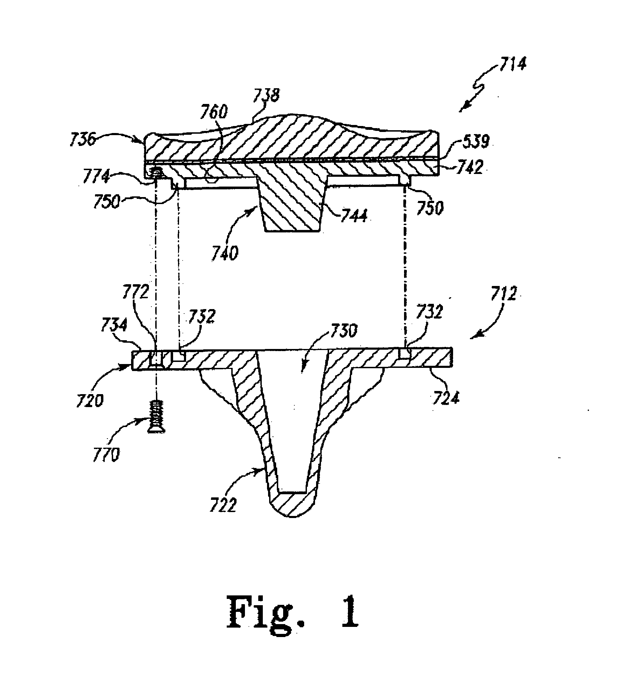

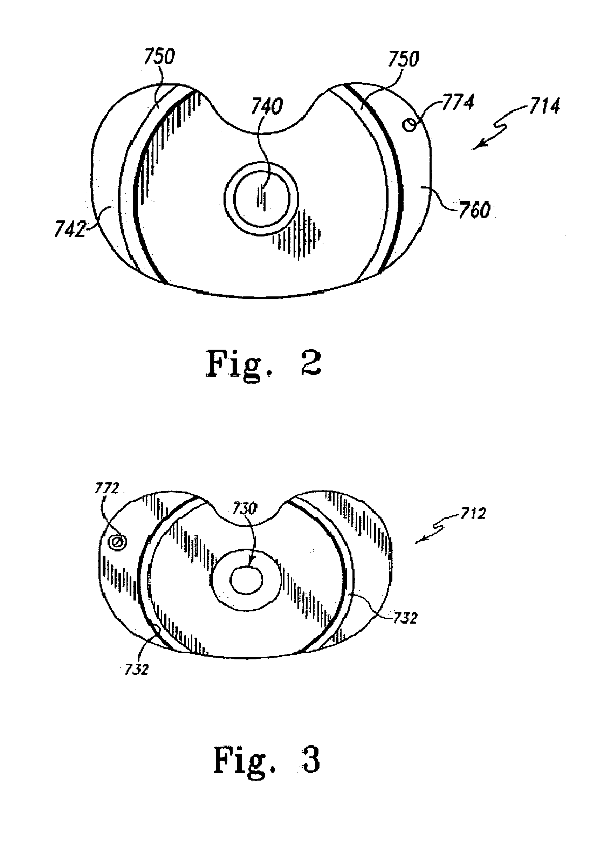

Referring to FIGS. 1 to 3, a prosthetic knee system includes a tibial tray 712 and a tibial insert 714. The tibial tray 712 includes a platform 720, a stem 722 coupled to the bottom surface 724 of the platform 720 and a bore 730 configured to receive a stem of the tibial insert 714 therein. The platform 720 includes a track defining grooves 732 formed in the upper surface 734 of the platform 720, as shown in FIGS. 1 and 3, for example. The tibial insert 714 includes a bearing portion 736 defining an upper bearing surface 738 and a stem portion 720 coupled to the bearing portion 736. The bearing portion 736 is made of a polymer such as UHMWPE, for example. The stem portion 740 includes a backing 742 coupled to the bearing portion 736 and a stem 744 coupled to the backing 742. The stem portion 740 is made from a metal such as titanium or cobalt chromium, for example. The stem portion 740 further includes two curved rails 750 coupled to the bottom surface of the backing 742 of the stem portion 740, as shown in FIGS. 1 and 2, for example.

In use, the stem 744 of the tibial insert 714 is received within the bore 730 of the tibial tray 712 and the rails 750 are each received within the corresponding grooves or tracks 732 formed in the platform 720 of the tibial tray 712 such that the bottom surface 760 of the platform 742 of the tibial insert 714 is adjacent to and engaged with the upper surface of the platform 720 of the tibial tray 712. In this configuration, the tibial insert 714 is able to rotate relative to the tibial tray 712 about a longitudinal axis through the stem 722 of the tibial tray 712. The rails 750 and the tracks 732 operate to guide and constrain such rotational movement of the insert 714 relative to the tray 712. In order to fix the tibial insert 714 relative to the tibial tray 712, a threaded screw 770 may be inserted through a countersunk bore 772 through the platform 720 of the tibial tray 712 and into a threaded bore 774 formed in the bottom surface 760 of the platform 742 of the tibial insert 714. As such, the tibial insert 714, the tibial tray 712, and the locking screw 770 cooperate to provide a non- rotating tibial assembly while the tibial insert 714 and the tibial tray 712 cooperate to provide a rotating tibial assembly, as discussed above.

As noted above, the stem portion 740 of the tibial insert 714 is made from metal such that the rail(s) 750 or 780 of the stem portion 740 is/are made from metal as well in order to slide within the corresponding metal tracks 732 of the tibial tray 712. Alternatively, the metal rail(s) 750 or 780 may be moulded directly into the bottom surface of the polymer bearing portion 736 of the insert 714 without the use of the metal backing 742 of the stem portion 740.

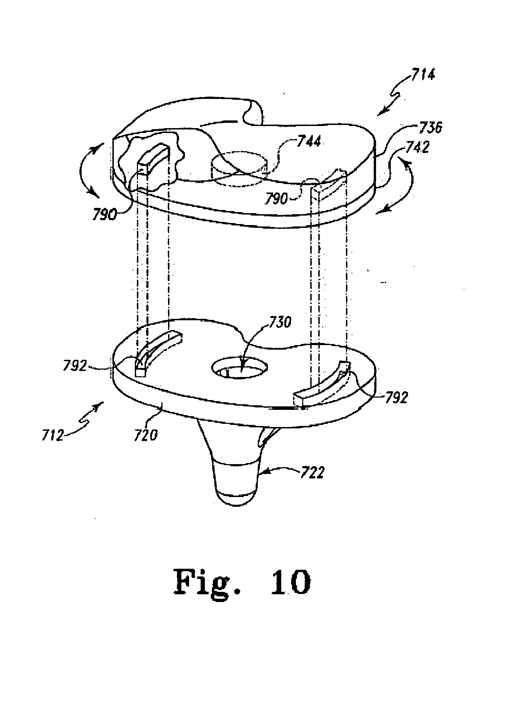

Looking now to FIG. 10, alternative rails or tabs 790 of the tibial insert 714 and alternative tracks or grooves 792 of the tibial tray 712 are provided. Similar to the rails 750 and corresponding grooves 732 described above in regards to FIGS. 1 to 3, the tabs 790 and tracks 792 operate to guide and constrain rotational movement of the insert 714 relative to the tray 721. Further, similar to that shown in FIG. 1, a locking screw 770 may be provided to fix the relative movement between the tibial insert 712 and the tray 714. Alternatively, the tabs 790 may have a tab or projection (not shown) to be received within an undercut feature (not shown) of the corresponding tracks 792 in order to prevent lift-off of the tibial insert 712 relative to the tray 714.

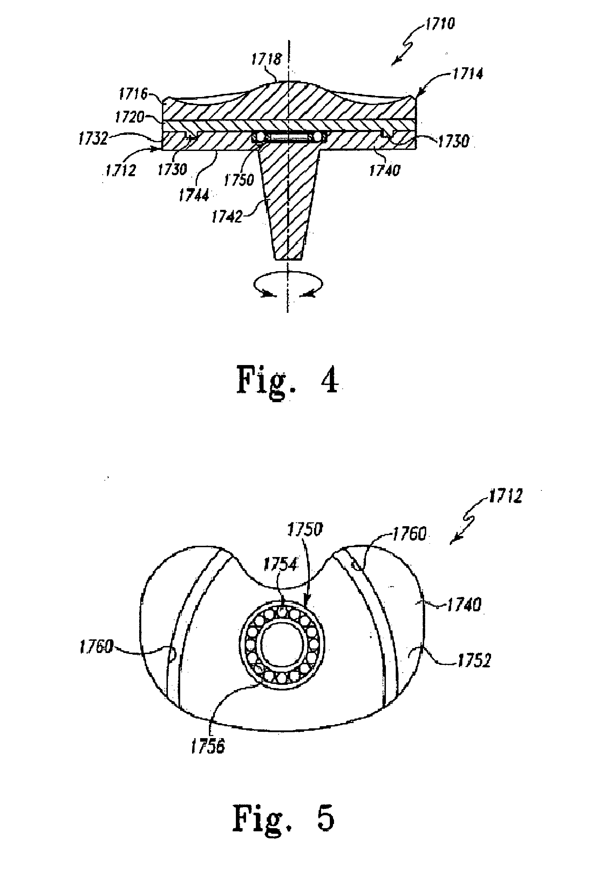

Looking now to FIGS. 4 and 5, a tibial assembly 1710 includes a tibial tray 1712 and a rotating tibial insert 1714. The rotating tibial insert 1714 includes a bearing portion 1716 defining an upper bearing surface 1718. The bearing portion 1716 is made of a polymer such as UHMWPE, for example. The tibial insert 1714 further includes a metal backing portion 1720 coupled to the bearing portion 1716 and includes a pair of rails 1730 extending downwardly from a bottom surface 1732 of the metal backing portion 1720.

The tibial tray 1712 includes a platform 1740 and a stem 1742 coupled to a bottom surface 1744 of the platform 1740. The platform 1740 further includes a roller bearing system 1750 incorporated into a top surface 1752 of the platform 1740. Illustratively, the roller bearing system 1750 includes a plurality of roller bearings 1754 set in a circular track 1756 coupled to the platform 1740. The platform 1740 further includes a pair of guide tracks 1760 formed to receive the downwardly- extending rails 1730 of the tibial insert 1714 therein. The roller bearings 1754 of the roller bearing system 1750 are metal and are adjacent to and engaged with the bottom surface 1732 of the metal backing portion 1720 of the tibial insert 1714. As such, the roller bearing system 1750 operates to decrease friction between the tibial insert 1714 and the tibial tray 1712 as the tibial insert 1714 is urged to rotate relative to the tibial tray 1714. The guide tracks 1760 and the rails 1730 cooperate to guide and constrain the rotational movement of the tibial insert 1714 relative to the tibial tray 1712.

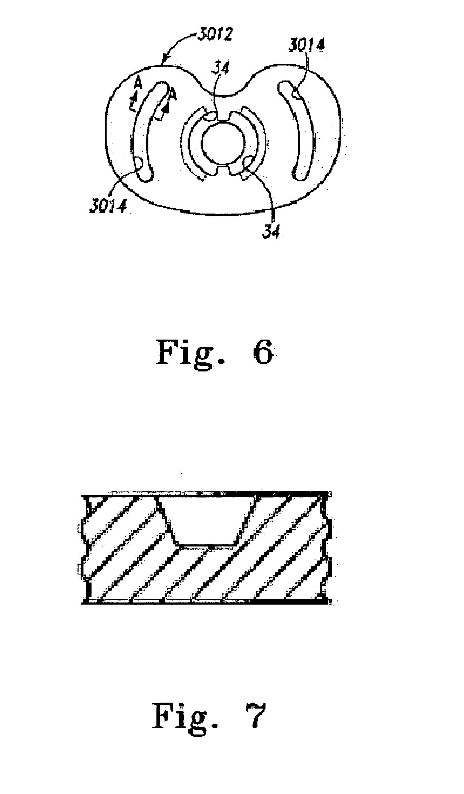

Looking now to FIGS. 6 to 9, a tray 3012 includes various cutout portions or slots, formed therein. For example, the cutout portions FIG. 6 include two curved, elongated openings 3014 independent from the recessed portions 34 of the tray 3012.

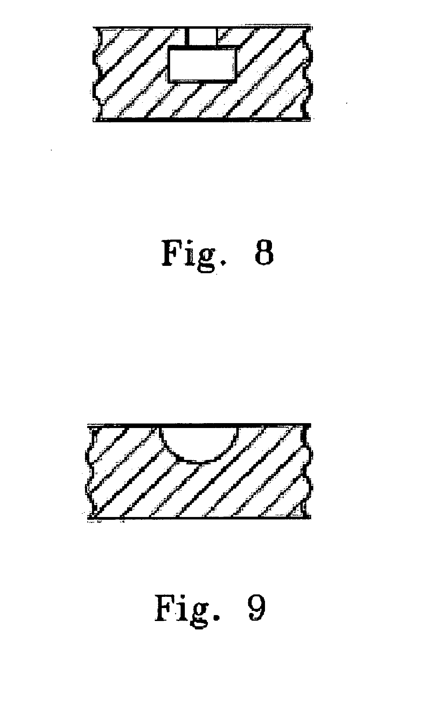

Looking specifically now to FIGS. 7 to 9, illustrative sectional views of the elongated openings 3014 shown in FIG. 6 are provided. In other words, each of the elongated openings 3014 may be formed to define any one of the cross-sectional profiles shown in FIGS. 7 to 9. For example, as shown in FIG. 7, the cross-section of any one of the elongated openings 3014 may tapered or trapezoidal in shape while the cross- section of any one of the cutout portions 3014 may be generally "T-shaped" as shown in FIG. 8, for example. Finally, the cross-section of any one of the cutout portions 3014 may simply be rounded, as shown in FIG. 9, and may illustratively be semi-circular.

## Claims

1. An orthopaedic prosthesis comprising:
a tibial tray (712) configured to be coupled to a surgically-prepared surface of the proximal end of a tibia, the tibial tray including a platform (720) having an upper surface (734), the platform including a curved first slot (732) and a curved second slot (732) defined in the upper surface, and
a tibial insert (714) coupled, in use, to the tibial tray, the tibial insert including (i) an upper bearing surface (738) configured for contacting a pair of femoral condyles, (ii) a bottom surface (760) for contacting the upper surface of the tibial tray, with a first curved tab (750) and a second curved tab (750) extending downwardly from the bottom surface, the first curved tab being received in the first curved slot of the tibial tray, and the second curved tab being received in the second curved slot of the tibial tray, when the bottom surface of the insert contacts the upper surface of the tray such that the tibial insert is able to rotate with respect to the tibial tray,
**characterised in that** the tibial insert includes a bearing portion (736) which provides the upper bearing surface (738), and a backing portion (740) which provides the bottom surface (760) and the first and second curved tabs (750), in which the bearing portion (736) is made from a polymer and the backing portion (740) is made from a metal.

2. The orthopaedic prosthesis of claim 1, in which each of the first and second curved slots (732) has a concave-shaped top profile.

3. The orthopaedic prosthesis of claim 1, in which the tibial insert (714) is configured to rotate with respect to the tibial tray (712) through less than 90°.

4. The orthopaedic prosthesis of claim 3, in which:
(i) the length of the first curved tab (750) is less than the length of the first curved slot (732), and
(ii) the length of the second curved tab (750) is less than the length of the second curved slot (732), in which the first curved tab is configured to slide along an first arc within the first curved slot and the second curved tab is configured to slide along a second arc within the second curved slot when the tibial tray (712) is rotated relative to the tibial insert (714).

5. The orthopaedic prosthesis of claim 1, in which:
(i) the tibial insert (714) includes a stem (744) extending downwardly from the bottom surface (760), and
(ii) the tibial tray (712) includes an opening (730) defined on the upper surface (734) of the platform (720) between the first slot (732) and the second slot (732), the stem of the tibial insert being received in the opening.

6. The orthopaedic prosthesis of claim 1, in which:
(i) the platform (720) of the tibial tray (712) includes a first bore defined through it, and
(ii) the tibial insert (714) includes a second bore defined in the bottom surface (760).

7. The orthopaedic prosthesis of claim 6, which includes a screw received in the first bore and the second bore to fix the tibial insert against movement with respect to the tibial tray.

8. The orthopaedic prosthesis of claim 7, in which the first bore is a countersunk bore and the second bore is a threaded bore.

## Patentansprüche

1. Orthopädische Prothese, umfassend:
- ein Tibiaplateau (712), das konfiguriert ist, um mit einer chirurgisch vorbereiteten Fläche des proxymalen Endes einer Tibia gekoppelt zu werden, wobei das Tibiaplateau eine Plattform (720) mit einer Oberseite (734) aufweist und die Plattform einen gekrümmten ersten Schlitz (732) und einen gekrümmten zweiten Schlitz (732) enthält, die in der Oberseite definiert sind, und
- einen Tibiaeinsatz (714), der im Gebrauch mit dem Tibiaplateau gekoppelt ist, wobei der Tibiaeinsatz (i) eine obere Lagerfläche (738), die zum Kontaktieren eines Paares Femurkondylen konfiguriert ist, und (ii) eine Unterseite (760) zum Kontaktieren der Oberseite des Tibiaplateaus enthält, wobei sich eine erste gekrümmte Nase (750) und eine zweite gekrümmte Nase (750) von der Unterseite nach unten erstrecken, wobei die erste gekrümmte Nase in dem ersten gekrümmten Schlitz des Tibiaplateaus aufgenommen wird und die zweite gekrümmte Nase in dem zweiten gekrümmten Schlitz aufgenommen wird, wenn die Unterseite des Einsatzes die Oberseite des Plateaus kontaktiert, so dass sich der Tibiaeinsatz in Bezug auf das Tibiaplateau drehen kann,
- **dadurch gekennzeichnet, dass** der Tibiaeinsatz ein Lagerteil (736), das die obere Lagerfläche (738) liefert, und ein Basisteil (740) enthält, das die Unterseite (760) und die ersten und zweiten gekrümmten Nasen (750) liefert, wobei das Lagerteil (736) aus einem Polymer hergestellt ist und das Basisteil (740) aus einem Metall hergestellt ist.

2. Orthopädische Prothese nach Anspruch 1, wobei jeder der ersten und zweiten gekrümmten Schlitze (732) ein konkaves Oberprofil aufweist.

3. Orthopädische Prothese nach Anspruch 1, wobei der Tibiaeinsatz (714) konfiguriert ist, um in Bezug auf das Tibiaplateau (712) um weniger als 90° zu drehen.

4. Orthopädische Prothese nach Anspruch 3, wobei:
(i) die Länge der ersten gekrümmten Nase (750) geringer als die Länge des ersten gekrümmten Schlitzes (732) ist und
(ii) die Länge der zweiten gekrümmten Nase (750) geringer als die Länge des zweiten gekrümmten Schlitzes (732) ist, wobei die erste gekrümmte Nase konfiguriert ist, um entlang eines ersten Bogens in dem ersten gekrümmten Schlitz zu gleiten, und die zweite gekrümmte Nase konfiguriert ist, um entlang eines zweiten Bogens in dem zweiten gekrümmten Schlitz zu gleiten, wenn das Tibiaplateau (712) relativ zum Tibiaeinsatz (714) gedreht wird.

5. Orthopädische Prothese nach Anspruch 1, wobei:
(i) der Tibiaeinsatz (714) einen Schaft (744) enthält, der sich von der Unterseite (760) nach unten erstreckt, und
(ii) das Tibiaplateau (712) eine Öffnung (730) enthält, die auf der Oberseite (734) der Plattform (720) zwischen dem ersten Schlitz (732) und dem zweiten Schlitz (732) definiert ist, wobei der Schaft des Tibiaeinsatzes in der Öffnung aufgenommen ist.

6. Orthopädische Prothese nach Anspruch 1, wobei:
(i) die Plattform (720) des Tibiaplateaus (712) eine erste Bohrung enthält, die durch sie hindurch gebildet ist, und
(ii) der Tibiaeinsatz (714) eine zweite Bohrung enthält, die in der Unterseite (760) gebildet ist.

7. Orthopädische Prothese nach Anspruch 6, die eine Schraube enthält, die in der ersten Bohrung und in der zweiten Bohrung aufgenommen ist, um den Tibiaeinsatz gegen eine Bewegung in Bezug auf das Tibiaplateau zu fixieren.

8. Orthopädische Prothese nach Anspruch 7, wobei die erste Bohrung eine Senkbohrung ist und die zweite Bohrung eine Gewindebohrung ist.

## Revendications

1. Prothèse orthopédique, comprenant :
un plateau tibial (712) configuré pour être couplé à une surface chirurgiquement préparée de l'extrémité proximale d'un tibia, le plateau tibial incluant une plateforme (720) ayant une surface supérieure (734), la plateforme incluant une première fente courbée (732) et une deuxième fente courbée (732) définies dans la surface supérieure, et
un insert tibial (714) couplé, en cours d'utilisation, au plateau tibial, l'insert tibial incluant (i) une surface de support supérieure (738) configurée pour venir en contact avec une paire de condyles fémoraux, (ii) une surface inférieure (760) destinée à venir en contact avec la surface supérieure du plateau tibial, avec une première patte courbée (750) et une deuxième patte courbée (750) s'étendant vers le bas à partir de la surface inférieure, la première patte courbée étant reçue dans la première fente courbée du plateau tibial, et la deuxième patte courbée étant reçue dans la deuxième fente courbée du plateau tibial, lorsque la surface inférieure de l'insert vient en contact avec la surface supérieure du plateau de sorte que l'insert tibial est apte à tourner relativement au plateau tibial,
**caractérisée en ce que** l'insert tibial comprend une portion de support (736) qui constitue la surface de support supérieure (738), et une portion d'endossage (740) qui constitue la surface inférieure (760), et les première et deuxième pattes courbées (750), la portion de support (736) étant réalisée en un polymère, et la portion d'endossage (740) est réalisée en un métal.

2. Prothèse orthopédique selon la revendication 1, dans laquelle chacune des première et deuxième fentes courbées (732) possède un profil de dessus de forme concave.

3. Prothèse orthopédique selon la revendication 1, dans laquelle l'insert tibial (714) est configuré pour tourner relativement au plateau tibial (712) selon moins que 90°.

4. Prothèse orthopédique selon la revendication 3, dans laquelle :
(i) la longueur de la première patte courbée (750) est plus petite que la longueur de la première fente courbée (732), et
(ii) la longueur de la deuxième patte courbée (750) est plus petite que la longueur de la deuxième fente courbée (732), où la première patte courbée est configurée pour coulisser le long d'un premier arc dans la première fente courbée, et la deuxième patte courbée est configurée pour coulisser le long d'un deuxième arc dans la deuxième fente courbée lorsque le plateau tibial (712) est amené à tourner relativement à l'insert tibial (714).

5. Prothèse orthopédique selon la revendication 1, dans laquelle :
(i) l'insert tibial (714) comprend une tige (744) s'étendant vers le bas depuis la surface inférieure (760), et
(ii) le plateau tibial (712) inclut une ouverture (730) définie sur la surface supérieure (734) de la plateforme (720) entre la première fente (732) et la deuxième fente (732), la tige de l'insert tibial étant reçue dans l'ouverture.

6. Prothèse orthopédique selon la revendication 1, dans laquelle
(i) la plateforme (720) du plateau tibial (712) inclut un premier perçage défini à travers celle-ci ; et
(ii) l'insert tibial (714) inclut un deuxième perçage défini dans la surface inférieure (760).

7. Prothèse orthopédique selon la revendication 6, qui comprend une vis reçue dans le premier perçage et le deuxième perçage pour immobiliser l'insert tibial à l'encontre d'un mouvement relativement au plateau tibial.

8. Prothèse orthopédique selon la revendication 7, dans laquelle le premier perçage est un perçage à contre-dépouille, et le deuxième perçage est un perçage taraudé.
